# EUROPEAN PATENT APPLICATION

(11) **EP 3 964 228 A1**
(43) Date of publication of application: **09.03.2022**
(21) Application number: 20802881.1
(22) Date of filing: 08.05.2020
(51) Int. Cl.: A61K 38/46, A61K 35/64, A61P 3/04, A23L 33/17, A23K 20/189

(54) **COMPOSITION FOR PREVENTING OR TREATING OBESITY, COMPRISING PHOSPHOLIPASE A2 AS ACTIVE INGREDIENT**

(30) Priority: 08.05.2019 KR 20190053866; 17.04.2020 KR 20200047003
(71) Applicant: UNIVERSITY-INDUSTRY COOPERATION GROUP OF KYUNG HEE UNIVERSITY, Yongin-si, Gyeonggi-do 17104 (KR)
(72) Inventor: BAE, Hyunsu, Seoul 05229 (KR); JEONG, Hyunju, Seoul 07638 (KR)
(74) Representative: Gassner, Birgitta
(86) International application number: PCT/KR2020/006089
(87) International publication number: WO 2020/226450

(57) **Abstract**

The present invention relates to a composition for improving obesity, comprising phospholipase A2, which is one of the main components of bee venom, as an active ingredient, and more specifically to: a pharmaceutical composition for preventing or treating obesity, comprising phospholipase A2 as an active ingredient; a functional health food composition for preventing or improving obesity, comprising phospholipase A2 as an active ingredient; a feed composition; a method for preventing or treating obesity, comprising a step of administering a composition comprising phospholipase A2 as an active ingredient to an individual; and a use, for preventing or treating obesity, of a composition comprising phospholipase A2 as an active ingredient. A composition for reducing inflammatory macrophages and for preventing or treating obesity, comprising phospholipase A2, which is a substance of the present invention and a major component of bee venom, as an active ingredient, can be usefully employed as a pharmaceutical composition.

<110>     University-Industry Cooperation Group of Kyung Hee University

<120>     Composition of preventing or treating obesity comprising
          phospholipase A2

<130>     OPA20081-PCT

<150>     KR 10-2019-0053866
<151>     2019-05-08

<160>     20

<170>     KoPatentIn 3.0

<210>     1
<211>     19
<212>     DNA
<213>     Artificial Sequence

<220>
<223>     GAPDH forward primer


<400>     1
cccagaagac tgtggatgg                                                    19


<210>     2
<211>     20
<212>     DNA
<213>     Artificial Sequence

<220>
<223>     GAPDH reverse primer


<400>     2
cacattgggg gtaggaacac                                                   20


<210>     3
<211>     32
<212>     DNA
<213>     Artificial Sequence

<220>
<223>     TNF-alpha forward primer


<400>     3
ttctgtctac tgaacttcgg ggtgatcggt cc                                     32


<210>     4
<211>     32
<212>     DNA
<213>     Artificial Sequence

<220>
<223>     TNF-alpha reverse primer


<400>     4

gtatgagata gcaaatcggc tgacggtgtg gg                                    32

<210>    5
<211>    27
<212>    DNA
<213>    Artificial Sequence

<220>
<223>    IL-1 beta forward primer

<400>    5
ggacagaata tcaaccaaca agtgata                                         27

<210>    6
<211>    23
<212>    DNA
<213>    Artificial Sequence

<220>
<223>    IL-1 beta reverse primer

<400>    6
gtgtgccgtc tttcattaca cag                                             23

<210>    7
<211>    20
<212>    DNA
<213>    Artificial Sequence

<220>
<223>    IL-12a forward primer

<400>    7
gctctagacc ctgtgccttg                                                 20

<210>    8
<211>    20
<212>    DNA
<213>    Artificial Sequence

<220>
<223>    IL-12a reverse primer

<400>    8
gaaggcttac ctgcatcagc                                                 20

<210>    9
<211>    18
<212>    DNA
<213>    Artificial Sequence

<220>
<223>    IL-4 forward primer

<400>    9
acgaagaaca ccacagag                                                    18


<210>    10
<211>    18
<212>    DNA
<213>    Artificial Sequence

<220>
<223>    IL-4 reverse primer


<400>    10
tgatgtggac ttggactc                                                    18


<210>    11
<211>    18
<212>    DNA
<213>    Artificial Sequence

<220>
<223>    CD206 forward primer


<400>    11
agtggcaggt ggcttatg                                                    18


<210>    12
<211>    18
<212>    DNA
<213>    Artificial Sequence

<220>
<223>    CD206 reverse primer


<400>    12
ggttcaggag ttgttgtg                                                    18


<210>    13
<211>    23
<212>    DNA
<213>    Artificial Sequence

<220>
<223>    Ym1 forward primer


<400>    13
cattcagtca gttatcagat tcc                                              23


<210>    14
<211>    18
<212>    DNA
<213>    Artificial Sequence

<220>

<223>    Ym1 reverse primer

<400>    14
agtgagtagc agccttgg                                                    18

<210>    15
<211>    20
<212>    DNA
<213>    Artificial Sequence

<220>
<223>    PPAR gamma forward primer

<400>    15
gaaggctgaa gtcaccaagc                                                  20

<210>    16
<211>    20
<212>    DNA
<213>    Artificial Sequence

<220>
<223>    PPAR gamma reverse primer

<400>    16
tcagccttgc cagagtttt t                                                 20

<210>    17
<211>    20
<212>    DNA
<213>    Artificial Sequence

<220>
<223>    C/EBP alpha forward primer

<400>    17
ttacaacagg ccaggtttcc                                                  20

<210>    18
<211>    20
<212>    DNA
<213>    Artificial Sequence

<220>
<223>    C/EBP alpha reverse primer

<400>    18
ctctgggatg gatcgattgt                                                  20

<210>    19
<211>    20
<212>    DNA
<213>    Artificial Sequence

<220>
<223>    UCP-1 forward primer

<400>    19
tctcagccgg cttaatgact                                                    20

<210>    20
<211>    20
<212>    DNA
<213>    Artificial Sequence

<220>
<223>    UCP-1 reverse primer

<400>    20
gctgggtgta tgtgcctttt                                                    20

## Description

### Technical Field

The present disclosure relates to a composition for amelioration of obesity containing as an active ingredient phospholipase A2, one of the main components of bee venom, and more specifically to a pharmaceutical composition for the prevention or treatment of obesity containing phospholipase A2 as an active ingredient, a health functional food composition for the prevention or amelioration of obesity containing phospholipase A2 as an active ingredient, a feed composition, a method for preventing or treating obesity including administering to a subject a composition containing phospholipase A2 as an active ingredient, and use of a composition containing phospholipase A2 as an active ingredient for the prevention or treatment of obesity.

### Background Art

Obesity, which is a representative symptom of excessive accumulation of fat in the body, is classified as one of the major metabolic diseases and is a serious health problem faced by countries around the world. At least 300 million people will be obese by 2025, and thus obesity drug markets are expected to grow greatly not only in Korea but also globally.

Obesity is known to cause diabetes, heart disease, high blood pressure, cancer, and the like in combination, and in severe cases, it results in early death.

Obesity is also classified as chronic inflammation, and recent studies have revealed that inflammatory macrophages accumulated in adipose tissue are closely associated with obesity. Moreover, the obesity condition creates an inflammatory environment due to an imbalance between inflammatory and anti-inflammatory macrophages in fat, and the infiltration of inflammatory macrophages increases the secretion of the inflammatory cytokine TNF-a or interleukin 6, aggravating the symptoms of obesity.

Although the alleviation of an inflammatory environment and the balance of macrophages are considered to be important for the treatment of obese patients, as of yet, there is no obesity therapeutic agent that targets macrophages.

Current obesity treatments include diet, exercise, and drug therapies. However, drug therapies have problems with respect to stability and side effects since the administration period increases, and thus there is a need for the development of novel obesity therapeutic agents.

At present, only orlistat and lorcaserine are approved for long-term use in Korea, and various therapeutic agents, such as liraglutide, phentermine/topiramate, and naltrexone/bupropion, have been developed and approved in the U.S. However, the development of fundamental obesity therapeutic drugs is urgent since many drugs decrease the body weight in a manner that interferes with the absorption of fat from foods.

Phospholipase A2 is a protein enzyme, and phospholipases, which are contained at a content of 12% in bee venom, are enzymes that hydrolyze phospholipids and are also called lecithinases. The phospholipase A2 component is known to cause cell tissue destruction, hemolysis, catalysis, and the like since it liberates fatty acids bonded to BDP of glycerin of phospholipids, which are cell membrane lipids, to form lysophosphatides. Various pharmaceutical compositions can be developed by utilizing such actions of phospholipase A2. Korean Patent Publication No. 10-2015-0049438 discloses a composition capable of preventing and treating viral diseases, such as influenza and bullous stomatitis. In addition, Korean Patent Publication No. 10-2012-0139313 discloses a composition containing a phospholipase as an active ingredient for treatment of a prion disease, wherein the composition can be helpfully used as a pharmaceutical composition for the prevention and treatment of a regenerative brain disease.

### Disclosure

### Technical Problem

The present inventors undertook intensive research efforts to develop methods for ameliorating obesity without side effects, and as a result, they have identified that phospholipase A2 derived from bee venom showed a preventive or therapeutic effect on obesity, thereby completing the present disclosure.

### Technical Solution

An aspect of the present invention is to provide a pharmaceutical composition for the prevention or treatment of obesity, the composition containing phospholipase A2 as an active ingredient.

Another aspect of the present invention is to provide a health functional food composition for the prevention or amelioration of obesity, the composition containing phospholipase A2 as an active ingredient.

Still another aspect of the present invention is to provide a feed composition for the prevention or amelioration of obesity, the composition containing phospholipase A2 as an active ingredient.

Still another aspect of the present invention is to provide a method for preventing or treating obesity, the method including administering to a subject a composition containing phospholipase A2 as an active ingredient.

Still another aspect of the present invention is to provide use of a composition containing phospholipase A2 as an active ingredient for the prevention or treatment of obesity.

### Advantageous Effects

The composition for the reduction of inflammatory macrophages and the prevention or treatment of obesity, the composition containing as an active ingredient phospholipase A2, which is a substance of the present invention and a major component of bee venom, can be helpfully used as a pharmaceutical composition.

### Brief Description of Drawings

FIG. 1A is a schematic diagram showing the experimental schedule.
FIG. 1B provides images showing conditions of mice by group.
FIG. 1C is a graph showing body weight changes of mice by group.
FIG. 1D is a graph showing food uptake of mice by group.
FIG. 1E provides images showing epididymal and inguinal white adipose tissues by group.
FIG. 1F provides images showing weight changes of epididymal and inguinal white adipose tissues by group.
FIG. 2A provides images showing conditions of CD206-deficient mice by group.
FIG. 2B is a graph showing body weight changes of mice by group.
FIG. 2C provides images showing epididymal and inguinal white adipose tissues by group.
FIG. 2D provides graphs showing weight changes of epididymal and inguinal white adipose tissues by group.
FIG. 2E is a graph showing body weight changes of CD206-deficient mice additionally treated with IgG by group.
FIG. 3A provides images showing livers of mice by group.
FIG. 3B is a graph showing liver weights of mice by group.
FIG. 3C provides H&E staining images of livers of mice by group.
FIG. 3D is a graph showing the lipid droplet accumulation in liver by group.
FIG. 3E provides H&E staining images of kidneys of mice by group.
FIG. 3F is a graph showing kidney hypertrophy of mice by group.
FIG. 3G provides graphs showing ALT, AST, BUN, and Crea levels in serum in mice by group.
FIG. 3H provides graphs showing TG, GLU, HDL-C, and LDL-C levels in serum in mice by group.
FIG. 3I provides graphs showing insulin and leptin levels in serum in mice by group.
FIG. 4A provides images showing macrophage infiltration in fat by group.
FIG. 4B provides graphs showing macrophage infiltration in fat by group.
FIG. 5A provides graphs showing TNF-a, IL-1β, and IL-12a levels by group through quantitative real-time PCR.
FIG. 5B provides graphs showing IL-4, CD206, and Ym1 levels by group through quantitative real-time PCR.
FIG. 5C provides graphs showing PPARγ, C/EBPa, and UCP-1 levels by group through quantitative real-time PCR.
FIG. 6 is a graph confirming body weight changes in mice administered with PLA2 and melittin.

### Detailed Description of the Invention

In accordance with one aspect of the present disclosure, there is provided a pharmaceutical composition for the prevention or treatment of obesity containing phospholipase A2 as an active ingredient.

As used herein, the term "phospholipase A2 (PLA2)" is one of the protein enzymes, wherein phospholipases contained in a content of 12% in bee venom are enzymes that hydrolyze phospholipids and are also called lecithinase. Phospholipases are categorized as A1, A2, B, C, and D according to the degradation site of phospholipids.

The obesity ameliorating efficacy of PLA2 was not known, and was first identified by the present inventors.

More specifically, PLA2 may be one (bvPLA2) derived from bee venom of *Apis mellifera* or one produced by way of genetic recombination (including genetic modification engineering), but is not limited thereto.

PLA2 of the present disclosure was identified to have a significantly excellent effect of inhibiting or treating obesity compared with melittin, a main component of bee venom.

In addition, the effects of PLA2 may be achieved by acting on mannose receptors, alleviating hepatic steatosis or kidney damage through the reduction of the accumulation of lipid droplets, alleviating metabolic dysfunction resulting from the reductions of aminotransferase, alanine aminotransferase, blood urea nitrogen, creatine, triglycerides (TG), glucose (GLU), high-density lipoprotein (HDL-C), or low-density lipoprotein (LDL-C), insulin, and leptin, by inhibiting the infiltration of pro-inflammatory macrophages, by controlling M1/M2 through a reduction of M1 macrophages or an increase of M2 macrophages, or by inhibiting lipid accumulation and lipid synthesis, but is not limited thereto.

As used herein, the term "obesity" refers to a state of an excess of fat in the body, and specifically, obesity is defined as a body mass index (calculated as weight (kg) divided by height (m) squared) of 25 or greater. Obesity may be caused by very complex causes, such as genetic and environmental factors, but consequentially, obesity is caused by the accumulation of fat due to energy imbalance occurring in cases of an excessive intake of nutrients relative to energy consumption over a long period of time. Obesity results in an increased likelihood of diabetes and hyperlipidemia and an increased risk of developing sexual dysfunction, arthritis, and cardiovascular disease.

As used herein, the term "prevention" may refer to any action that inhibits or delays obesity by administration of, to a subject, the composition containing PLA2 as an active ingredient according to the present disclosure.

As used herein, the term "treatment" may refer to any action that favorably or advantageously changes obesity symptoms by administration of the composition of the present disclosure to a subject suspected of obesity.

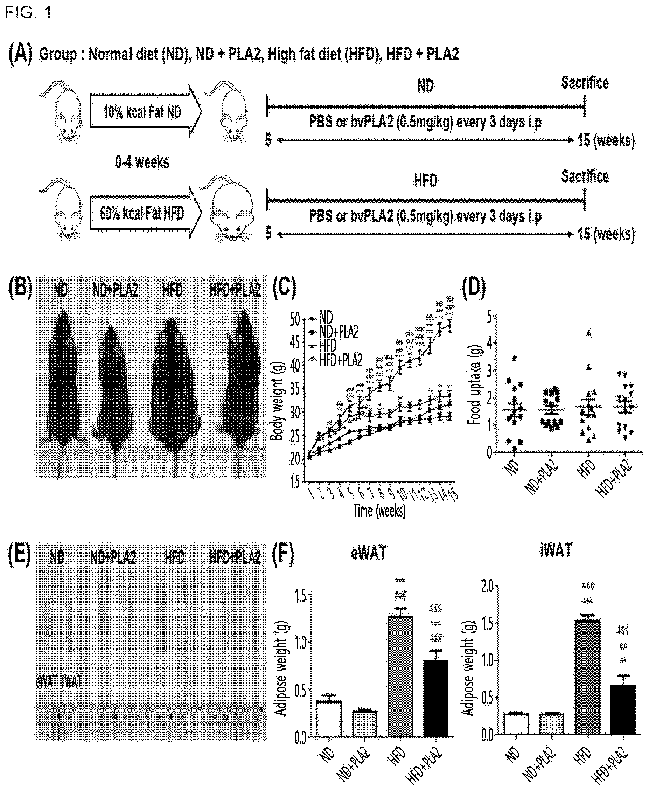

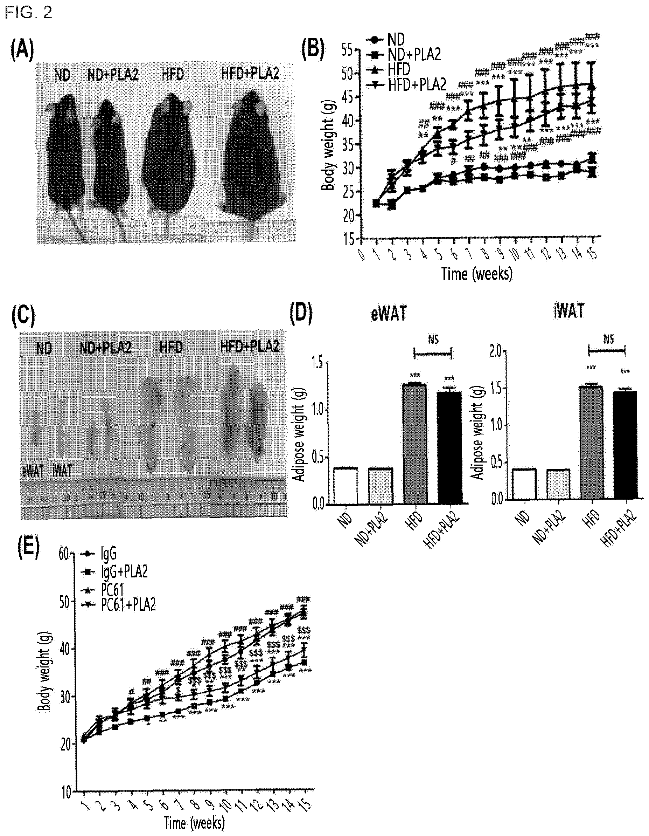

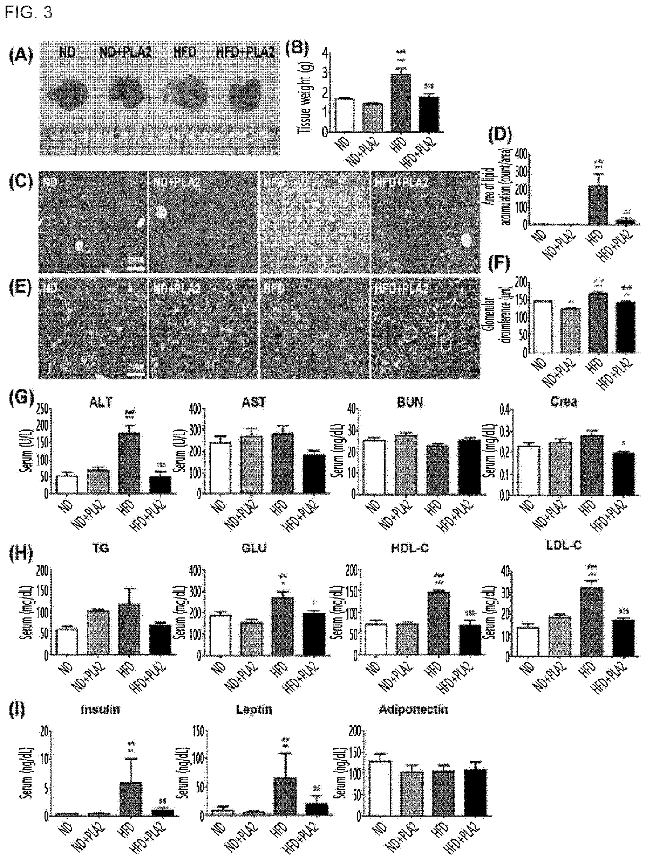

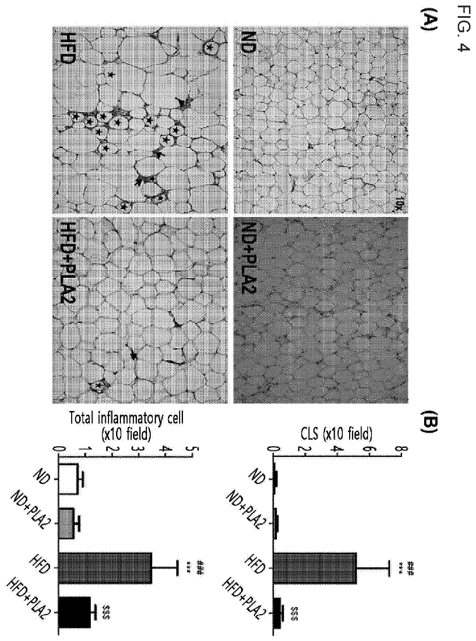

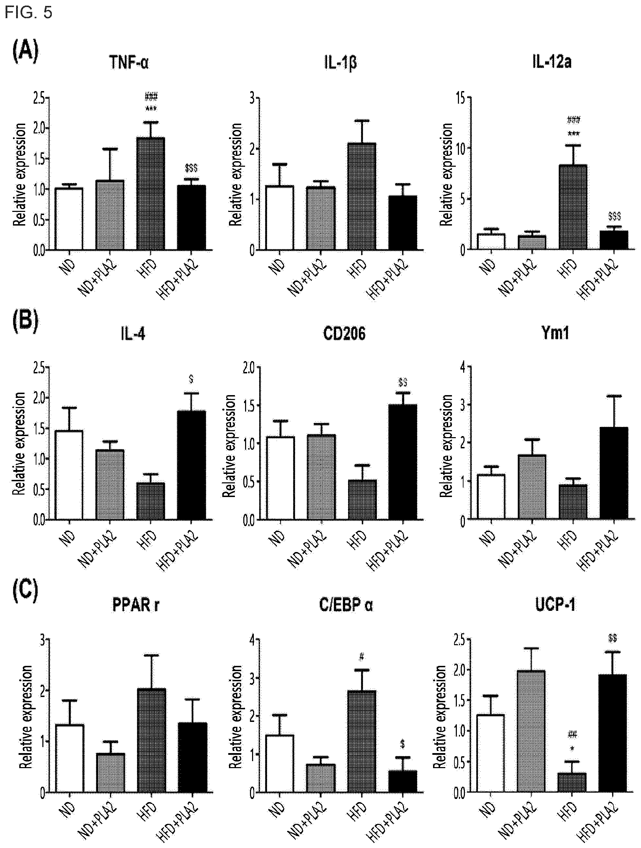

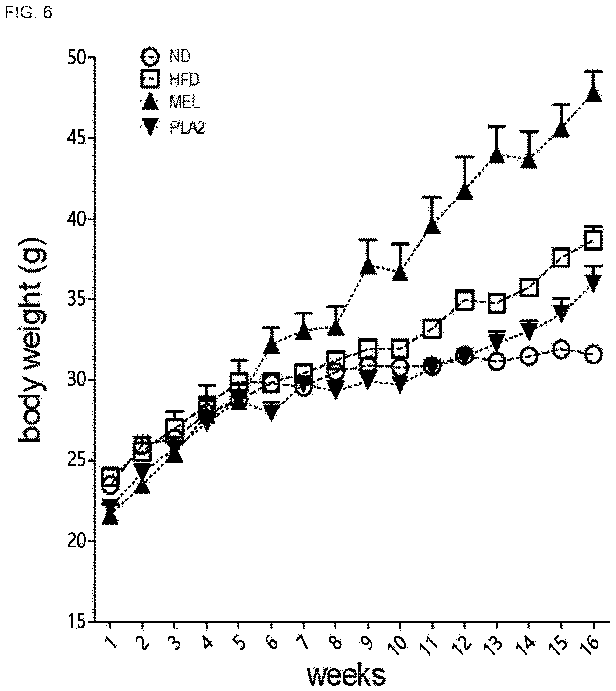

In exemplary embodiments of the present disclosure, it was identified through the administration of PLA2 to mice that PLA2 had effects of reducing adipose tissue weight and body weight (FIG. 1); it was identified through mice lacking the mannose receptor that the obesity inhibitory effect of bvPLA2 resulted from the mannose receptor (FIG. 2); it was identified through H&E staining that PLA2 had effects of alleviating hepatic steatosis and kidney inflammation due to reduction of lipid droplet accumulation, and it was identified through serum examination of mice for each group that PLA2 had an effect of alleviating metabolic dysfunction (FIG. 3); it was identified through biopsy that PLA2 had an effect of ameliorating obesity by inhibiting macrophage infiltration, and it was identified through M1/M2 macrophage marker analysis that PLA2 modulated the M1/M2 macrophage ratio (FIG. 4) and modulated adipogenic factor expressions (FIG. 5), and thus had an effect of inhibiting or treating obesity; and it was identified PLA2 showed an excellent obesity inhibitory effect compared with melittin, a main component of bee venom (FIG. 6), thus completing the present disclosure.

The pharmaceutical composition of the present disclosure may be used as a single formulation, or may be prepared as a complex formulation by further including a drug known to have approved obesity treatment effects. The pharmaceutical composition may be formulated using a pharmaceutically acceptable carrier or excipient and may be prepared as a unit dosage form or contained in a multi-dose container.

As used herein, the term "pharmaceutically acceptable carrier" refers to a carrier or a diluent that does not inhibit biological activity and characteristics of a compound to be injected while causing no irritation to an organism. The carrier usable in the present disclosure is not particularly limited to the kind thereof, and any carrier may be used as long as it is commonly used in the art and is pharmaceutically acceptable. Non-limiting examples of the carrier may include a saline solution, sterile water, Ringer's solution, buffered saline, an albumin infusion solution, a dextrose solution, a maltodextrin solution, glycerol, ethanol, and the like. These may be used alone or in a mixture of two or more thereof. The carrier may include a carrier which does not occur naturally.

In addition, as necessary, other common additives, such as an antioxidant, a buffer, and/or a bacteriostatic agent, may be added and used, and a diluent, a dispersant, a surfactant, a binder, a lubricant, and the like may be further added to be formulated as an injectable formulation, such as an aqueous solution, a suspension, or an emulsion, a pill, a capsule, granules, a tablet, or the like and used.

In addition, the pharmaceutical composition of the present disclosure may contain a pharmaceutically effective amount of PLA2.

As used herein, the term "pharmaceutically effective amount" refers to an amount sufficient to treat diseases at a reasonable benefit/risk ratio applicable to medical treatment, wherein, generally, an amount of 0.001 mg/kg to 1000 mg/kg, preferably an amount of 0.05 mg/kg to 200 mg/kg, and more preferably an amount of 0.1 mg/kg to 100 mg/kg may be administered once to several times a day. However, for the purpose of the present disclosure, it is preferable that a specific therapeutically effective amount for a certain patient is differently applied according to various factors, including the types and extents of reactions to be achieved, the specific composition including whether another preparation is used in accordance with circumstances, the age, weight, usual physical condition, sex, and diet of a patient, the time of administration, the route of administration, the secretion rate of the composition, the duration of treatment, and a drug used together with or simultaneously with the specific composition, and similar factors well known in the field of medicine.

The pharmaceutical composition of the present disclosure may be administered as an individual therapeutic agent or in combination with another therapeutic agent, and may be administered sequentially or simultaneously with conventional therapeutic agents. In addition, single or multiple dosing may be performed. It is important that administration be performed considering all of the aforementioned factors so that a maximum effect can be obtained with a minimum amount without any side effects, and such an amount may be easily determined by a person skilled in the art.

As used herein, the term "administration" refers to an introduction of the pharmaceutical composition of the present disclosure into a patient by way of any suitable method. The composition of the present disclosure may be administered through various routes of oral or parenteral administration as long as the composition can reach a target tissue.

The manner of administration of the pharmaceutical composition of the present disclosure is not particularly limited, and may follow methods that are commonly used in the art. A non-limiting example of the manner of administration may be an oral administration or parenteral administration of a composition. The pharmaceutical composition according to the present disclosure may be prepared as various formulations depending on the desired manner of administration.

The frequency of administration of the composition of the present disclosure is not particularly limited, but may be once daily or divided into several doses.

In accordance with another aspect of the present disclosure, there is provided a health functional food composition for the prevention or amelioration of obesity containing phospholipase A2 as an active ingredient.

In accordance with still another aspect of the present disclosure, there is provided a feed composition for the prevention or amelioration of obesity containing phospholipase A2 as an active ingredient.

As used herein, the terms "phospholipase A2", "obesity", and "prevention" are as described above.

As used herein, the term "amelioration" refers to any action that at least reduces the extent of a parameter, for example, a symptom, associated with the condition to be treated, by way of the administration of a composition containing PLA2 as an active ingredient.

As used herein, the term "health functional food", which is the same term as a food for special 24-8 health use (FoSHU), refers to a food with high medicinal and medical effects to efficiently exhibit a bio-regulatory function in addition to a function of nutrient supply, wherein the food may be prepared in various forms, such as a tablet, a capsule, a powder, granules, a liquid, and a pill, to obtain useful effects in the prevention or amelioration of obesity.

The food composition of the present disclosure may further contain a sitologically acceptable carrier.

The type of food to which the composition containing PLA2 of the present disclosure can be added is not particularly limited, and examples thereof are various drinks, gums, teas, vitamin complexes, health supplement foods, and the like. The food composition may additionally contain other ingredients that do not interfere with the prevention or amelioration of obesity, and the type of ingredient is not particularly limited.

For example, the food composition, like common foods, may contain as additional ingredients, various kinds of herbal extracts, sitologically acceptable food supplement additives, natural carbohydrates, or the like.

The food supplement additives are added to manufacture respective formulations of health functional foods, and may be appropriately selected by a person skilled in the art. For example, examples of the food supplement additive may include several types of nutrients, vitamins, minerals (electrolytes), flavoring agents, such as synthetic flavoring agents and natural flavoring agents, coloring agents, fillers, pectic acid and salts thereof, alginic acid and salts thereof, organic acids, protective colloid thickeners, pH adjusters, stabilizers, preservatives, glycerin, alcohol, carbonating agents used for carbonated drinks, and the like, but the type of the food supplement additive is not limited to the examples.

The content of an extract contained in the food may be, but is not particularly limited to, 0.01 wt% to 100 wt%, more preferably 1 wt% to 80 wt%, relative to the total weight of the food composition.

When the food is a drink, the extract may be contained at an amount of 1 g to 30 g, preferably 3 g to 20 g, relative to 100 mL of the drink. The composition may further contain additional ingredients that are commonly used in a food composition to enhance flavor, taste, visual appearance, and the like. For example, the composition may contain vitamins A, C, D, E, B1, B2, B6, and B12, niacin, biotin, folate, pantothenic acid, and the like. The composition may also contain food additives, for example, minerals, such as zinc (Zn), iron (Fe), calcium (Ca), chromium (Cr), magnesium (Mg), manganese (Mn), and copper (Cu). The composition may also contain amino acids, such as lysine, tryptophan, cysteine, and valine. The composition may also contain antiseptics (potassium sorbate, sodium benzoate, salicylic acid, sodium dehydroacetate, *etc.),* disinfecting agents (bleaching powder and high-grade bleaching powder, sodium hypochlorite, *etc.),* antioxidants (butylhydroxyanisole (BHA), butylhydroxytoluene (BHT), *etc.),* colorants (tar dyes, *etc.),* color formers (sodium nitrite, *etc.),* bleaching agents (sodium sulfite), seasoning agents (MSG, sodium glutamate, *etc.),* sweeteners (dulcin, cyclamate, saccharine, sodium, *etc.),* flavoring agents (vanillin, lactones, *etc.),* blowing agents (alum, potassium D-bitartrate, *etc.),* fortifying agents, emulsifying agents, viscosity-increasing agents (thickening agents), coating agents, gum bases, anti-foaming agents, solvents, modifiers, and the like. The additives may be selected according to the type of food, and may be used in suitable amounts.

The health functional food of the present disclosure can be manufactured by way of a method that is commonly used in the art, and can be manufactured by adding raw materials and ingredients that are commonly added in the art. Unlike common drugs, the health functional food of the present disclosure may be prepared using a food as a raw material, and thus has advantages of avoiding side effects that may occur due to long-term administration of drugs and having excellent portability.

In accordance with still another aspect of the present disclosure, there is provided a method for preventing or treating obesity, the method including administering to a subject the composition containing phospholipase A2 as an active ingredient of the present disclosure.

As used herein, the term "subject" may refer to any animal, including a human, who has been obese or is likely to be obese. The animal may be not only a human but also a mammal, such as a cow, a horse, a sheep, a pig, a goat, a camel, an antelope, a dog, or a cat, in need of treatment for a symptom similar to that of the human, but is not limited thereto.

Specifically, the preventing or treating method of the present disclosure may include administering the composition at a pharmaceutically effective amount to a subject that is obese or is likely to be obese.

As used herein, the term "administration" refers to an introduction of the pharmaceutical composition of the present disclosure into a patient by way of any suitable method. The composition of the present disclosure may be administered through various routes of oral or parenteral administration as long as the composition can reach a target tissue.

In accordance with still another aspect of the present disclosure, there is provided use of the composition of the present disclosure for the prevention or treatment of obesity, the composition containing phospholipase A2 as an active ingredient.

### Mode for Carrying Out the Invention

Hereinafter, constitutions and effects of the present disclosure will be described in detail with reference to exemplary embodiments. However, these exemplary embodiments are used only for illustrating the present disclosure, and the scope of the present disclosure is not limited by these examples.

### Test Example 1: Increase in anti-inflammatory macrophages by bvPLA2

Bee venom-derived phospholipase A2 (bvPLA2, *Apis mellifera*)*,* 3-isobutyl-1-methylxanthine (IBMX), dexamethasone (DEX), insulin, and oil red O were purchased from Sigma-Aldrich (St. Louis, MO, USA).

### Test Example 2: Alleviation of inflammatory environment by bvPLA2

3T3-L1 pre-adipocytes were purchased from the Korea Cell Line Bank (KCLB, Seoul, Korea) and grown in DMEM supplemented with 10% bovine calf serum (BCS), and the culture medium was exchanged every two or three days. Thereafter, the cells were seeded in a 96-well plate, and the medium was exchanged with MDI (DMEM containing IBMX, 111 µg/mL; DEX, 2 µM; and insulin, 2 µg/mL) to induce adipocyte differentiation. The differentiation medium was exchanged every two days, and after 4 days of differentiation, fully differentiated cells were analyzed through oil red O staining.

### Test Example 3: Oil red O staining

The differentiated 3T3-L1 cells were washed two times with PBS and fixed with a 4% paraformaldehyde buffer for 20 minutes. Oil red O (Sigma-Aldrich St. Louis, MO, USA) was dissolved in 60% isopropanol, and then used to stain the cells by treatment at room temperature for 20 minutes. The cells were washed two times with distilled water, and then the OD value was measured at 510 nm.

### Test Example 4: Experimental animals

Male C57BL/6 mice (5 weeks old; weight 18-20 g) and CD206-/- mice (B6.129P2-MRC1tm1Mnz/J) were purchased from Jackson Laboratory (Bar Harbor, ME). After acclimation for one week, the mice were fed a normal diet (ND, 10 kcal%) or a high-fat diet (HFD, 60 kcal%) for 15 weeks. Thereafter, the mice were intraperitoneally injected with PBS or bvPLA2 (0.5 mg/kg) starting on week 5 and continuing for 11 weeks.

### Test Example 5: Regulatory T cell depletion (Treg depletion)

Anti-mouse CD25 (clone: PC61) was produced from hybridomas obtained from the American Type Culture Collection (ATCC; Manassas, VA, USA), and the mice were intraperitoneally injected with anti-mouse CD25 or rat IgG (Sigma-Aldrich) every 3 days for 11 weeks.

### Test Example 6: Quantitative real-time PCR

Total RNA was isolated from white adipose tissue (WAT) samples by using an easy-BLUE RNA extraction kit (iNtRON Biotechnology, Korea), and cDNA was synthesized using Cyclescript reverse transcriptase (Bioneer, Korea). The synthesized cDNA was subjected to quantitative real-time PCR (95°C for 15 sec, 55°C for 10 sec, and 72°C for 10 sec) with SensiFAST SYBR no-Rox kit (Bioline, Korea), and each reaction was performed in triplicate. The primer sequences for the experiment were as follows:
GAPDH:
   forward, 5'-CCCAGAAGACTGTGGATGG-3'
   reverse, 5'- CACATTGGGGGTAGGAACAC-3'
TNF-a:
   forward, 5'-TTCTGTCTACTGAACTTCGGGGTGATCGGTCC-3'
   reverse, 5'- GTATGAGATAGCAAATCGGCTGACGGTGTGGG-3'
IL-1β:
   forward, 5'-GGACAGAATATCAACCAACAAGTGATA-3'
   reverse, 5'-GTGTGCCGTCTTTCATTACACAG-3'
IL-12a:
   forward, 5'-GCTCTAGACCCTGTGCCTTG-3'
   reverse, 5'-GAAGGCTTACCTGCA TCAGC-3'
IL-4:
   forward, 5'-ACGAAGAACACCACAGAG-3'
   reverse, 5'-TGATGTGGACTTGGACTC-3'
CD206:
   forward, 5'-AGTGGCAGGTGGCTTATG-3'
   reverse, 5'-GGTTCAGGAGTTGTTGTG-3'
Ym1:
   forward, 5'-CATTCAGTCAGTTATCAGATTCC-3'
   reverse, 5'-AGTGAGTAGCAGCCTTGG-3'
PPARγ:
   forward, 5'-GAAGGCTGAAGTCACCAAGC-3'
   reverse, 5'-TCAGCCTTGCCAGAGTTTTT-3'
C/EBPa:
   forward, 5'-TTACAACAGGCCAGGTTTCC-3'
   reverse, 5'-CTCTGGGATGGATCGATTGT-3'
UCP-1:
   forward, 5'-TCTCAGCCGGCTTAATGACT-3'
   reverse, 5'-GCTGGGTGTATGTGCCTTTT-3'

### Test Example 7: H&E staining

Liver and kidney tissues were fixed in 4% paraformaldehyde overnight, dehydrated, embedded in paraffin, and sectioned to a thickness of 4 µm. The sections were deparaffinized with xylene and then hydrated again with 100%, 90%, 80%, and 70% ethanol. The hydrated sections were washed with tap water and stained with a hematoxylin solution for 5 minutes. Thereafter, the stained sections were immersed in 1% acid alcohol, and then stained again in an eosin solution for 3 minutes. Thereafter, for mounting, the stained sections were dehydrated with 70%, 80%, 90%, or 100% ethanol to remove xylene.

### Test Example 8: Statistical analysis

The test results were expressed as the mean and standard error, and statistical significance was analyzed by one-way ANOVA and the Newman-Keuls test using Prism 5 software.

### Example 1: Effects of bvPLA2 of reducing adipose tissue weight and body weight

The obesity amelioration effect of bvPLA2 was evaluated through changes in adipose tissue weight and body weight of mice with HFD-induced obesity.

More specifically, the C57BL/6 mice were fed ND or HFD for 15 weeks. After 4 weeks of ND or HFD feeding, the mice were injected with PLA2 or PBS, starting on week 5 (FIG. 1A). Thereafter, the adipose tissue and body weight were measured according to the injection or non-injection of bvPLA2.

As a result, as shown in FIG. 1, the body weight of the HFD group was higher than that of the ND group, and the HFD+PLA2 group showed a significant reduction in body weight compared with the HFD group without PLA2 administration (FIGS. 1B and 1C). However, no food uptake change was observed in all of the groups (FIG. 1D). In addition, the epididymal WAT and inguinal WAT weights were significantly increased in the HFD group, but greatly reduced in the HFD+PLA2 group (FIGS. 1E and 1F).

These results confirmed that the administration of bvPLA2 significantly reduced the body weight and the adipose tissue weight without influencing food uptake, suggesting that bvPLA2 has an obesity ameliorating effect.

### Example 2: Influence of bvPLA2 on body weight and adipose tissue weight of CD206-deficient and regulatory T cell-deficient mice

In order to investigate the immunoregulatory effect of bvPLA2, CD206-deficient (CD206-/-) mice lacking the mannose receptor were used.

More specifically, the body weight and adipose tissue weight of CD206-/- mice were measured by way of the same method as the above experimental schedule (FIG. 1A).

As a result, as shown in FIG. 2, there were no significant changes in body weight and adipose tissue weight between the HFD and HFD+PLA2 groups (FIGS. 2A to 2D).

In order to investigate the correlation between bvPLA2 and regulatory T cells, regulatory T cell depletion was performed by injecting anti-mouse CD25 antibody (clone PC61) into mice every 3 days. Rat IgG was used as a control.

As a result, as shown in FIG. 2, the body weight was significantly reduced in the IgG+PLA2 obesity-induced mouse group compared with the IgG-only obesity-induced mouse group. The body weight was also markedly reduced in the PC61+PLA2 group compared with the PC61-treated group (FIG. 2E).

These results confirmed that PLA2 showed an obesity inhibitory effect through the mannose receptor, but there was no difference between the IgG+PLA2 and PC61+PLA2 groups, suggesting that the obesity inhibitory effect of PLA2 is not correlated with regulatory T cells mediated by PC61.

### Example 3: Hepatotoxicity/nephrotoxicity reducing effects of bvPLA2

### Example 3-1: Effects of bvPLA2 of ameliorating hepatic steatosis and kidney damage

In order to investigate whether PLA2 alleviated HFD-induced hepatic steatosis and kidney inflammation, liver and kidney tissues from the mice were H&E stained.

As a result, as shown in FIGS. 3A to 3D, the size and weight of the liver were observed to increase due to lipid droplet accumulation and hepatomegaly in the HFD group, compared with the ND group. However, such increases were attenuated in the bvPLA2-treated HFD group, and no influence was observed in the ND+PLA2 group.

Next, the glomerular diameter indicating an early indicator of diabetic nephropathy caused by kidney damage was investigated.

As a result, as shown in FIGS. 3E and 3F, kidney hypertrophy caused by lipid accumulation was observed in the HFD group, but no kidney hypertrophy (increased glomerular diameter) was observed in the ND, ND+PLA2, and HFD+PLA2 groups (FIGS. 3E and 3F).

These results confirmed that bvPLA2 alleviated hepatic steatosis and kidney damage by reducing lipid droplet accumulation.

### Example 3-2: Metabolic dysfunction alleviating effect of bvPLA2

The effects of bvPLA2 on liver and kidney functions were investigated.

More specifically, serum levels of alanine aminotransferase (ALT), aminotransferase (AST), blood urea nitrogen (BUN), and creatine (Crea) were measured for each group.

As a result, as shown in FIG. 3G, the AST, ALT, and Crea levels were increased in the HFD-administered group compared with the ND group. However, the increased levels of AST, ALT, and Crea were reduced in the bvPLA2-treated HFD group.

In order to investigate the effect of bvPLA2 on metabolic syndrome, the triglyceride (TG), glucose (GLU), high-density lipoprotein cholesterol (HDL-C), and low-density lipoprotein cholesterol (LDL-C) levels were analyzed for each group.

As a result, as shown in FIG. 3H, the TG, GLU, HDL-C, and LDL-C levels were markedly higher in the HFD group compared with the ND group. However, the increased levels were significantly reduced in the HFD+PLA2 group.

In addition, plasma insulin and leptin levels were analyzed for each group.

As a result, as shown in FIG. 31, the plasma insulin and leptin levels were increased in the HFD group compared with the ND group, and the increased levels were significantly reduced in the HFD+PLA2 group (FIG. 31).

These results confirmed that bvPLA2 alleviated the HFD-induced metabolic dysfunction.

### Example 4: Effect of bvPLA2 of inhibiting macrophage infiltration in adipose tissue

Since obesity is characterized by the localization of crown-like structures (CLS), which are formed by pro-inflammatory macrophages surrounding dead adipocytes in AT28, H&E histological examination was performed to investigate whether bvPLA2 inhibited the macrophage infiltration in WAT.

As a result, as shown in FIG. 4, the ND group showed few infiltrating pro-inflammatory macrophages in WAT, but the HFD group showed a significant increase in CLS formation. However, the bvPLA2-treated HFD+PLA2 group showed reduced macrophage infiltration and CLS formation in AT.

These results confirmed that bvPLA2 had an obesity ameliorating effect by inhibiting the infiltration of pro-inflammatory macrophages causing obesity.

### Example 5: Effects of bvPLA2 of modulating M1 or M2 phenotype markers and adipogenic factors

The changes in macrophage phenotypes were analyzed by quantitative real-time PCR analysis.

More specifically, the expressions of TNF-a, IL-1β, and IL-12a were measured to investigate the effect of bvPLA2 on M1-related markers.

As a result, as shown in FIG. 5A, TNF-a, IL-1β, and IL-12a expressions were significantly increased in the HFD group compared with the ND group, but in the HFD+PLA2 group, the increased TNF-a, IL-1β, and IL-12a expressions were inhibited to levels similar to those in ND (FIG. 5A).

In addition, the expressions of the M2-related markers IL-4, CD206, and Ym1 were measured.

As a result, as shown in FIG. 5B, the IL-4 and CD206 expressions were reduced in the HFD group compared with the NF group, but the reduced expressions were significantly increased in the HFD-PLA2 group (FIG. 5B).

In addition, the expressions of PPARy, C/EBPa, and UCP-1, which are markers related with lipid accumulation and adipogenesis, were measured.

As a result, as shown in FIG. 5C, the PPARy and C/EBPa expressions were increased in the HFD group compared with the ND group, but such increases were inhibited in the HFD+PLA2 group. However, the expression of UCP-1 was significantly reduced in the HFD group compared with the ND group, but significantly increased in the HFD+PLA2 group.

These results confirmed that the HFD conditions increased the M1-related markers and decreased the M2-related markers and also changed the expressions of adipogenesis-related markers, but bvPLA2 suppressed such changes, leading to significant improvement.

### Example 6: Comparison with melittin in bee venom

In order to investigate a component having an excellent obesity inhibitory effect among the components of bee venom, melittin, which is the most abundant in bee venom, was compared with bvPLA2, which is PLA2 derived from bee venom.

More specifically, the mice of the ND and HFD groups were intraperitoneally administered with PBS, melittin, and bvPLA2 at 0.5 mg/kg each for 16 weeks, and then the body weight changes were compared every day.

As a result, as shown in FIG. 6, the melittin-administered HFD group actually showed an increase in body weight compared to the control group without melittin administration, but the PLA2-administered HFD group showed a decrease in body weight compared with the control group.

These results confirmed that melittin, a main component of bee venom, had no obesity inhibitory effect, and that the PLA2 component had an obesity inhibitory effect.

While the present disclosure has been described with reference to the particular illustrative embodiments, a person skilled in the art to which the present disclosure pertains can understand that the present disclosure may be embodied in other specific forms without departing from the technical spirit or essential characteristics thereof. Therefore, the examples described above should be construed as exemplifying and not limiting the present disclosure. The scope of the present disclosure is not defined by the detailed description set forth above but by the accompanying claims of the disclosure, and it should also be understood that all changes or modifications derived from the definitions and scopes of the claims and their equivalents fall within the scope of the disclosure.

## Claims

1. A pharmaceutical composition for the prevention or treatment of obesity, the composition comprising phospholipase A2 as an active ingredient.

2. The composition of claim 1, wherein the prevention or treatment of obesity by phospholipase A2 is achieved by way of inhibition of the infiltration of pro-inflammatory macrophages.

3. The composition of claim 1, wherein the prevention or treatment of obesity by phospholipase A2 is achieved by a reduction of M1 macrophages or an increase of M2 macrophages.

4. The composition of claim 1, wherein the prevention or treatment of obesity by phospholipase A2 is achieved by way of alleviation of hepatic steatosis or kidney damage.

5. The composition of claim 1, wherein the prevention or treatment of obesity by phospholipase A2 is achieved by way of alleviation of metabolic dysfunction.

6. The composition of claim 1, further comprising a pharmaceutically acceptable carrier.

7. The composition of claim 1, wherein the phospholipase A2 is derived from bee venom or produced by way of genetic recombination.

8. The composition of claim 13, wherein the bee venom is derived from *Apis mellifera.*

9. A health functional food composition for the prevention or amelioration of obesity, the composition comprising phospholipase A2 as an active ingredient.

10. A feed composition for the prevention or amelioration of obesity, the composition comprising phospholipase A2 as an active ingredient.

11. A method for preventing or treating obesity, the method comprising administering to a subject a composition comprising phospholipase A2 as an active ingredient.

12. Use of a composition comprising phospholipase A2 as an active ingredient for the prevention or treatment of obesity.
